# EUROPEAN PATENT SPECIFICATION

(11) **EP 0 359 260 B1**
(45) Date of publication and mention of the grant of the patent: **21.04.1993**
(21) Application number: 89117032.6
(22) Date of filing: 14.09.1989
(51) Int. Cl.: A61K 31/66

(54) **The use of inositoltrisphosphate for the preparing of a medicament against abnormal levels of lipoproteins**
Verwendung von Inositoltriphosphat zur Herstellung eines Arzneimittels gegen abnorme Konzentrationen von Lipoproteinen
Utilisation du triphosphate d'inositol pour préparer un médicament contre les taux anormaux des lipoprotéines

(30) Priority: 15.09.1988 SE 8803248
(43) Date of publication of application: 21.03.1990
(73) Proprietor: PERSTORP AB, 284 80 Perstorp (SE)
(72) Inventor: Sirén, Matti, CH-6926 Montagnola Lugano (CH)
(74) Representative: Hansen, Bernd, Dr. Dipl.-Chem.

(56) References cited:
- EP-A- 0 179 439
- MED. KLIN., vol. 73, no. 7, 1978, pages 231-239; W. SCHWARTZKOPFF et al.: "Zur Kombinations- oder Monotherapie der Hyperlipoproteinämien Typ IIb, IV, V mit Clofibrat und m-Inositolnicotinat oder Clofibrinsäure"
- DIABETES, vol. 37, no. 11, November 1988, pages 1542-1548; H. XIANG et al.: "Effect of myo-inositol and T3 on myocardial lipids and cardiac function in Streptozocin-induced diabetic rats"

## Description

The present invention relates to the use of at least one isomer of inositoltrisphosphate for the preparing of a medicament for preventing, regulating or combatting abnormal levels of lipoproteins in mammals including man.

Disorders of lipoprotein metabolism are related to abnormalities in the synthesis and degradation of plasma lipoproteins. These abnormalities may result from primary inborn errors of metabolism or may be secondary to a variety of other conditions such as diabetes mellitus, hypothyroidism, excessive intake of alcohol and obesity.

Hyperlipoproteinemias are conditions in which the concentration of cholesterol- or triglyceride-carrying lipoproteins in plasma exceeds an arbitrary normal limit often defined as the ninety-fifth percentile of a random population.

Elevated concentrations of lipoproteins are considered to accelerate the development of arteriosclerosis, thrombosis and infarctions.

Many different types of lipoproteins exist such as chylomicrons, very-low-density lipoproteins (VLDL), low-density lipoproteins (LDL) and high-density lipoproteins (HDL), which contain different amounts of triglycerides, cholesterol or derivatives thereof.

The principle of treatment of all hyperlipoproteinemias is the provision of a diet that maintains a normal body weight and decreases the concentration of lipids in plasma.

However, this treatment is in many cases not sufficient and therefore different types of drugs are used.

Nicotinic acid has a limited use due to its side effects, such as gastrointestinal disturbances and hepatic disfunctions.
Clofibrate is another drug used in this area but in order to give positive effect the dosage must be high. Furthermore an increased risk is considered for patients with coronary diseases to get a worse condition when treated with this drug.
Other used drugs are gemfibrozil and cholestyramine. The clinical experience with the former is relatively limited and its long time safety is not established. The latter one, which binds bile acids in the intestine is quite unpleasant for the patients as the required dosage is high.

Despite the existence of different types of drugs in this area there is a need for an effective treatment without any adverse effects.

According to the present invention it has surprisingly become possible to fullfil the above mentioned needs by using at least one isomer of inositoltrisphosphate (IP₃) for the preparing of a medicament for preventing, regulating and combatting abnormal levels of lipoproteins in mammals including man.

From the European Patent No. 179439 a pharmaceutical composition comprising as a pharmaceutically active ingredient at least one isomer of inositoltrisphosphate is known. In said patent the effect of this pharmaceutical composition is shown for different areas such as cardiovascular diseases.

The medicament according to the invention can be used against abnormal levels of lipoproteins such as increased levels of cholesterol or derivatives thereof, increased levels of triglycerides and conditions related to increased levels of chylomicrons, LDL or VLDL. The abnormal levels could be related to plasma concentrations but also to the amount of lipoproteins in tissues.

The production of IP₃ and the isolation of the different isomers thereof are disclosed in the US patent No 4.777.134. The IP₃ isomers can also be produced by synthetic methods, chemically or enzymatically, starting with e.g. inositol and a phosphorus source. Furthermore, microbiological production methods including hybrid DNA-techniques of IP₃ are also suitable.

The structure of IP₃ and the different isomers thereof are disclosed in the US patent No 4.735.936 and the US patent No 4.797.390.

It is suitable that the medicament used according to the invention exists in unit dosage form. Tablets, granules or capsules are suitable administration forms for such unit dosage. Furthermore, tablets and granules can easily be surface treated such as to provide an enteric coating to prevent an uncontrolled hydrolysis in the stomach and to bring about a desired absorption in the intestine. Other suitable administration forms are slow release and transdermal administration. A usual pharmaceutically acceptable additive, excipient and/or carrier can be included in the medicament. The tablets or granules can also contain a disintegrant which causes the tablets or the granules, respectively, to disintegrate easily in the intestine. In certain cases, especially in acute situations, it is preferable to use the unit dosage in the form of a solution for intravenous administration.

The medicament can also consist as such of IP₃ solely without any additive, excipient or carrier.

If desired, the medicament can be free of other inositol phosphates, IP₁, IP₂, IP₄, IP₅ and IP₆. Accordingly, the mixture of IP₃ isomers can have a purity of 90-100%, such as 93-100% or preferably 95-100%.

Alternatively, the medicament can consist of or comprise one or more specific IP₃ isomers, each present in substantially pure form. Thus, the different isomers can be isolated from each other in substantially pure form, which means that they have a purity of 80-100%, such as 82-100% or 85-100%, preferably 90-100%. Since the isomers can be produced in pure form they can be mixed in any proportion, of course.

The medicament can consist of IP₃, wherein said IP₃ is provided by at least one of IP₆, IP₅ or IP₄ and a degradative substance such as an enzyme suitable to form IP₃.

It is in most cases suitable that the IP₃-isomer or isomers used for the preparing of the medicament according to the invention is present in salt form in order not to affect the mineral balance negatively. The salt should preferably consist of a sodium, potassium, calcium, zinc or magnesium salt or a mixture of two or more of these salts. Calcium and zinc salts or mixtures of these are especially preferred. The isomer of IP₃ can also partly be present as a salt of one or more physiologically acceptable compounds in the lanthanide series.

For the above mentioned reasons it is also an advantage if the medicament contains a surplus or an extra addition of at least one pharmaceutically acceptable salt of calcium, zinc or magnesium with a mineral acid or organic acid. This is especially valuable for elderly persons who are often deficient in these minerals.

For administration to human patients appropriate dosages can routinely be determined by those skilled in this art by extension of the results obtained in animals at various dosages. The preferred dosage for humans falls within the range of 0.1 to 1000 mg, especially 0.1-200 mg IP₃/day/kg body weight.

In animal experiments, no toxic effects were seen after administration of very high doses of IP₃, 160 mg/kg body weight by intraperitoneal injection to mice.

The medicament usually contains 0.01-1.5 g, such as 0.05-1.3 or preferably 0.1-1 g of IP₃ per unit dosage.

In one preferred embodiment of the invention the isomer of IP₃ is D-myo-inositol-1.2.6-trisphosphate.

One type of function of the medicament is to reverse, prevent or alleviate damage to membranes of different cell types, but especially cell membranes of platelets, erythrocytes and endothelial cells. The use of the medicament results in an improved stabilization, a decreased susceptibility to deformation and an improved function of the cell types. Furthermore, parameters such as aggregability and adhesion to e.g. vessel walls are decreased. The use of the medicament affects the levels and the activities of certain enzymes such as alkaline phosphatase. Furthermore a normalization of the inositol metabolism is obtained when the medicament is used. Other results of the use of the medicament are regulation of membrane fluidity, the incorporation of components such as cholesterol and the production, incorporation and balance between different phospholipids.

Another result of the use of the medicament is the regulation of the electrolyte balance of the cell. As examples, regulation of intracellular and extracellular levels of calcium, potassium, chloride and phosphate can be mentioned.

The cell surface activity and responsiveness to external stimuli via receptor activation /deactivation due to for example phosphorylation are other parameters effected by the use of the medicament.

The invention will be further explained with the following examples. Examples 1-4 show the production of IP₃ whereas examples 5 and 6 illustrate the preparation of solutions and tablets of IP₃. Examples 7-9 relate to the effect of IP₃ on different conditions of abnormal levels of lipoproteins.

### Example 1

### Hydrolysis of sodium phytate with baker's yeast and fractionation of a mixture of inositol phosphates.

A 1.4 kg quantity of sodium phytate (from corn, Sigma Chemical Co) was dissolved in 1 200 l sodium acetate buffer pH 4.6. 100 kg of baker's yeast from Jästbolaget, Sweden (dry substance: 28 %, nitrogen content: 2 %, phosphorus content: 0.4 %) was added with stirring and incubation was continued at 45°C. The dephosphorylation was followed by determining the inorganic phosphorus released. After 7 hours when 50 % inorganic phosphorus was liberated the hydrolysis was stopped by adding ammonia to pH 12. The suspension was centrifuged and the supernatant was collected.

800 l of the supernatant was passed through an ion-exchange column (Dowex 1, chloride form, 100 cm x 150 cm) and eluted with a linear gradient of hydrochloric acid (0-0.7 N HCL).

### Example 2

### Structural determination of isomers of inositol trisphosphate.

The fraction obtained in example 1 with a phosphorus/ inositol ratio of three to one was neutralized and precipitated as a calcium salt. 100 mg of the precipitate was analyzed with H-NMR. Data show that the peak consists of myo-inositol-1.2.6-trisphosphate.

### Example 3

A 0.5 gram quantity of D-chiro-inositol was dissolved in 1 ml phosphoric acid at 60°C. 20 g polyphosphoric acid was added and the mixture was heated to 150°C under vacuum for 6 hours. The mixture was diluted with water to a volume of 200 ml and passed through an ion-exchange column (Dowex 1, chloride form, 25 mm x 250 mm) and eluted with a linear gradient of hydrochloric acid (0-2.0 N HCl).

The content of the peak with the ratio of phosphorus to inositol of six to one was precipitated by addition of calciumhydroxide. The precipitate was filtered, washed and mixed with 10 ml of a cation-exchange resin to give the acid form of the inositolhexaphosphate. After neutralization with sodium hydroxide and freeze-drying the sodium salt of D-chiro-inositolhexaphosphate was obtained.

### Example 4

A 0.8 gram quantity of the sodium salt of D-chiro-inositolhexaphosphate produced according to Example 3 was dissolved in 300 ml sodium acetate buffer, pH 5.2. 1.3 gram wheat phytase (EC 3.1.3.26, 0.015 U/mg from Sigma Chemical Co.) was added and the mixture was incubated at 38°C.

After the liberation of 50 % inorganic phosphorus the hydrolysis was stopped by adding ammonia to pH 12.

The mixture containing D-chiro-inositolphosphates was passed through an ion-exchange column (Dowex 1 chloride form, 25 mm x 250 mm) and eluted with a linear gradient of hydrochloric acid (0-0.7 N HCL).

The peak with the ratio of phosphorus to inositol of three to one was neutralized with 1.0 M sodium hydroxide and freeze-dried.

Structural determination with NMR and IR showed the product to be D-chiro-inositoltrisphosphate.

### Example 5

### Solution of sodium salt of D-myo-inositol-1.2.6-trisphosphate for injection.

0.5 g of the sodium salt of IP₃ and 0.77 g NaCl were dissolved in 98.73 ml of water for injection to form a solution suitable for injection into a person or an animal.

### Example 6

### Tablets of calcium salt of D-myo-inositol-1.2.6-trisphosphate.

Tablets of the calcium salt of D-myo-inositol-1.2.6-trisphosphate were produced in the following way. 50 g calcium salt of D-myo-inositol-1.2.6-trisphosphate, 132 g lactose and 6 g acacia were mixed. Purified water was then added to the mixture, whereupon the mixing was continued until a suitable consistency was obtained. The mixture was sieved and dried. Then the mixture was blended with 10 g talcum and 2 g magnesium stearate. The mixture was compressed into tablets each weighing 200 mg.

### Example 7

An increased level of HDL and decreased levels of e.g. β-lipoproteins are beneficial characteristics for an effective treatment of abnormal levels of lipoproteins.

Thirty male rats were fed a standard diet supplemented with 2% cholesterol and 1% cholic acid for a 14-day pretreatment period. During the subsequent 14-day treatment period one group of rats was given the above diet with 0.2% D-myo-inositol-1.2.6-trisphosphate (IP₃) added, while the other rats continued on the pretreatment diet and served as a control. The effect on serum concentration of lipid- and cholesterolmetabolites is summarized below (%):

| Treatment | Total cholesterol | HDL cholesterol | β-lipo protein |
|---|---|---|---|
| Control | 100 | 100 | 100 |
| IP₃ | 78 | 116 | 82 |

Thus, it can be seen that the levels of cholesterol and β-lipoprotein is decreased, while the level of HDL-cholesterol is increased in the animals treated with IP₃. These data show that IP₃ has a beneficial effect on the lipoproteins in the body.

### Example 8

Injection of streptozotocin into rats results in an increase of lipidcomponents in plasma. 19 male rats weighing approximately 300g each were divided into two groups.
On the first day of the experiment both group 1 (8 animals) and group 2 (11 animals) received intraperitoneal injections of streptozotocin (Sigma) diluted in sodium citrate.
The first week both groups were fed a normal laboratory diet followed by a diet comprising casein, starch, glucose and fructose, butter and different oils. Starting from the second week group 2 also received D-myo-inositol-1.2.6-trisphosphate (IP₃, 160 mg/kg diet) mixed in the diet.

After eight weeks the animals were fasted for 6 hours and blood was removed from the jugular vein.
Triglycerides were determined and the results are shown in the following table:

| Treatment | Triglycerides (mg/dl) |
|---|---|
| Control, group 1 | 1535 |
| IP₃, group 2 | 1071 |

As can be seen the IP₃-treatment (group 2) markedly decreases the level of triglycerides.

### Example 9

A diet comprising a large fraction butter induces increased levels of cholesterol both in plasma and aorta.

36 rabbits, weighing approximately 2800g each, were divided into two groups.
Both group 1 (19 animals) and group 2 (17 animals) received a diet comprising 25% casein, 33% sucrose, 1.5% vitamin mixture, 2.5% mineral salts, 8% cellulose, 12% sawdust and 18% butter. Group 2 also received D-myo-inositol-1.2.6-trisphosphate (IP₃, 200 mg/kg diet) mixed in the diet.

After six months blood samples were taken before the animals were killed. Autopsies from aorta were extracted in order to determine the amount of cholesterol. Determined concentrations of cholesterol in plasma and aorta are shown below:

| Treatment | Cholesterol, serum mg/dl | Cholesterol, aorta mg/g |
|---|---|---|
| Control, group 1 | 290 | 4.5 |
| IP₃, group 2 | 245 | 3.5 |

The IP₃-treatment decreases cholesterol levels in serum and also the amount of cholesterol incorporated in the aorta.

## Claims

1. The use of at least one isomer of inositoltrisphosphate (IP₃) for the preparing of a medicament for preventing, regulating or combatting abnormal levels of lipoproteins in mammals including man.

2. The use according to claim 1, wherein the abnormal levels of lipoproteins are related to triglycerides.

3. The use according to claim 1, wherein the abnormal levels of lipoproteins are related to cholesterol.

4. The use according to any one of claims 1-3, wherein said inositoltrisphosphate is in salt form.

5. The use according to claim 4, wherein said inositoltrisphosphate salt is a salt of sodium, potassium, calcium or zinc.

6. The use according to any on of claims 1-5, wherein the medicament is in tablet or granulated form.

7. The use according to any one of claims 1-5, wherein the medicament is in the form of a solution.

8. The use according to any one of claims 1-5, wherein said inositoltrisphosphate is D-myo-inositol-1.2.6-trisphosphate.

## Patentansprüche

1. Verwendung von zumindest einem Isomer von Inositoltriphosphat (IP₃) zur Herstellung eines Medikamentes zur Verhinderung, Regulierung oder Linderung von abnormalen Gehalten von Lipoproteinen in Säugern, einschließlich den Menschen.

2. Verwendung nach Anspruch 1,
dadurch **gekennzeichnet,** daß
die abnormalen Gehalte an Lipoproteinen mit Triglyceriden zusammenhängen.

3. Verwendung nach Anspruch 1,
dadurch **gekennzeichnet,** daß
die abnormalen Gehalte an Lipoproteinen mit Cholesterin zusammenhängen.

4. Verwendung nach einem der Ansprüche 1 bis 3,
dadurch **gekennzeichnet,** daß
das Inositoltriphosphat in Salzform vorliegt.

5. Verwendung nach Anspruch 4,
dadurch **gekennzeichnet,** daß
das Inositoltriphosphatsalz ein Salz von Natrium Kalium, Kalzium oder Zink ist.

6. Verwendung nach einem der Ansprüche 1 bis 5,
dadurch **gekennzeichnet,** daß
das Medikament in Tablettenform oder granulierter Form vorliegt.

7. Verwendung nach einem der Ansprüche 1 bis 5,
dadurch **gekennzeichnet,** daß
das Medikament in der Form einer Lösung vorliegt.

8. Verwendung nach einem der Ansprüche 1 bis 5,
dadurch **gekennzeichnet,** daß
das Inositoltriphosphat D-Myo-inositol-1,2,6-triphosphat ist.

## Revendications

1. Utilisation d'au moins un isomère du trisphosphate d'inositol (IP₃) pour la préparation d'un médicament pour la prévention, la régulation ou la lutte contre des taux anormaux de lipoprotéines chez les mammifères, y compris l'homme.

2. Utilisation selon la revendication 1, dans laquelle les taux anormaux de lipoprotéines sont liés aux triglycérides.

3. Utilisation selon la revendication 1, dans laquelle les taux anormaux de lipoprotéines sont liés au cholestérol.

4. Utilisation selon l'une quelconque des revendications 1 à 3, dans laquelle ce trisphosphate d'inositol est sous forme de sel.

5. Utilisation selon la revendication 4, dans laquelle ce sel du trisphosphate d'inositol est un sel de sodium, de potassium, de calcium ou de zinc.

6. Utilisation selon l'une quelconque des revendications 1 à 5, dans laquelle le médicament est sous forme de comprimés ou sous forme granulée.

7. Utilisation selon l'une quelconque des revendications 1 à 5, dans laquelle le médicament est sous la forme d'une solution.

8. Utilisation selon l'une quelconque des revendications 1 à 5, dans laquelle ce trisphosphate d'inositol est le 1,2,6-trisphosphate de D-myo-inositol.
